# EUROPÄISCHE PATENTANMELDUNG

(11) **EP 4 564 004 A1**
(43) Veröffentlichungstag der Anmeldung: **04.06.2025**
(21) Anmeldenummer: 23212842.1
(22) Anmeldetag: 28.11.2023
(51) Int. Cl.: G01N 33/543, B01L 3/00

(54) **VERFAHREN ZUR BESTIMMUNG DER HYDROPHOBIZITÄT VON PROTEINEN**

(71) Anmelder: PAIA Biotech GmbH, 51105, Köln (DE)
(72) Erfinder: Aris, Perrou, 51105 Köln (DE)
(74) Vertreter: Zehetner, Andrea Christine

(57) **Zusammenfassung**

Verfahren zur Analyse von Proteinen unterschiedlicher Hydrophobizität mittels Spektroskopie oder unter Einsatz eines Biosensor, umfassend die Verwendung von Mikrotiterplatten, in deren Vertiefungen (Wells) bei der Analyse zumindest teilweise unterschiedliche Salz-Endkonzentrationen vorhanden sind, deren Abstände im Bereich von 10 bis 500 mMol/L liegen, und unter Verwendung von hydrophob-funktionalisierten Oberflächen.

## Beschreibung

Die Anmeldung betrifft ein Verfahren zur Analyse von Proteinen mit unterschiedlicher Hydrophobizität unter Verwendung von Mikrotiterplatten, deren Vertiefungen (Wells) zumindest teilweise unterschiedliche Salzkonzentrationen aufweisen und unter Verwendung von funktionalisierten Oberflächen.

Der Herstellung von Proteinen und insbesondere der Herstellung und Entwicklung von Biopharmazeutika einschließlich Viruspartikel-basierter Therapeutika und Antikörper-Wirkstoff-Konjugate (AWK) kommt große wirtschaftliche Bedeutung zu. Die Herstellung der Proteine und insbesondere von Biopharmazeutika ist jedoch sehr aufwendig und nimmt viel Zeit in Anspruch. Biopharmazeutika sind hinsichtlich ihrer physikalischen Eigenschaften heterogener als chemisch hergestellte Arzneistoffe, sog. niedermolekulare Arzneistoffe (Small Molecule Drugs).

Biopharmazeutika einschließlich Viruspartikel-basierter Therapeutika sind im Allgemeinen pharmazeutisch nutzbare und wirksame Proteine, wie z.B. Antikörper und Virenpartikel sowie Antikörper-Wirkstoff-Konjugate (AWK).

Bei der Herstellung und Handhabung von Proteinen und insbesondere von Biopharmazeutika können durch Fehlsynthesen, Degeneration oder andere Reaktionen unerwünschte Nebenprodukte entstehen, die identifiziert und abgetrennt werden müssen, da diese häufig die biologische Wirksamkeit des Zielproteins beeinflussen.

Bei den Nebenprodukten handelt es sich meist um fehlerhaft assemblierte, z.B. um fehlgepaarte (mispaired) Proteine, bei denen sich die verschiedenen Proteindomänen nicht so assembliert haben wie vorgesehen, oder um Fragmente des Zielproteins.

Unterschiede bei Biopharmazeutika, insbesondere bei Antikörpern, Fc-Fusionsproteinen, Antikörperfragmenten, bi- und multispezifischen Antikörpern sowie Viruspartikel-basierten Therapeutika und Antikörper-Wirkstoff-Konjugaten (AWK) können dazu führen, dass sich die Proteine in einem Biopharmazeutikum z.B. in ihrer Struktur, Stabilität, Bindungsaffinität unterscheiden, was die therapeutische Wirksamkeit des Biopharmazeutikums beeinflussen kann.

Bei der Herstellung von Proteinen, insbesondere von Biopharmazeutika ist es daher wichtig, Zelllinien und Kultivierungsbedingungen zu finden, bei denen möglichst wenige fehlerhafte Assemblierungen, Fragmente und Proteinfaltungen auftreten. In diesem sog. Upstream-Development fallen oft hunderte von Proben an, die in kurzer Zeit auf ihre Eignung untersucht werden müssen.

Gleiches gilt für das sogenannte Downstream-Development, in dem die Aufreinigung der Proteine, insbesondere Biopharmazeutika optimiert wird.

Mispairing tritt z.B. auf, wenn nach der Expression von bi- oder multispezifischen Antikörpern die produzierten unterschiedlichen Aminosäureketten nicht in der gewünschten Weise zusammengesetzt werden. Trotz verschiedener Ansätze, die Assemblierung zu optimieren und in Richtung des gewünschten Zielproteins zu verschieben, stellt dieses Mispairing ein großes Problem dar.

Proteine, die unterschiedliche Hydrophobizität aufweisen, sind auch unterschiedlich mit pharmazeutisch wirksamen Substanzen konjugierte Antikörper, sogenannte Antikörper-Wirkstoff-Konjugate (AWK) in Englisch: antibody drug conjugates (ADCs), wenn sie aufgrund ihrer unterschiedlichen Konjugation (Anzahl und Art der konjugierten Substanzen, Konjugationsstelle im Antikörper und Art der verwendeten Linker) unterschiedlich hydrophob sind.

Oberstes Ziel bei der Entwicklung von Verfahren zur Herstellung und Aufreinigung von Proteinen, insbesondere Biopharmazeutika sollte es daher sein, Bedingungen zu finden, unter denen möglichst wenig Nebenprodukte entstehen. Dies muss experimentell ermittelt werden und erfordert eine engmaschige Kontrolle des Versuchsansatzes (z.B. der Zellkultur), um zeitnah Änderungen der Versuchsbedingungen vornehmen zu können.

Auch Virenpartikel, die zum Beispiel in der Gentherapie eingesetzt werden, können unterschiedlich geladen und/oder ein unterschiedliches Maß an Hydrophobizität aufweisen. Die Beladung der Virenpartikel mit Nukleinsäuren (DNA oder RNA) führt beispielsweise dazu, dass die Viruspartikeloberfläche stärker negativ geladen ist als bei nicht oder nur teilweise beladenen Viruspartikeln. Gleichfalls ändert sich das Maß an Hydrophobizität je nach Beladungszustand. Da die Beladung der Viruspartikel mit den Nukleinsäuren essenziell für die therapeutische Wirkung ist, ist es sehr wichtig auch in Produktionsprozessen von Viruspartikeln darauf zu achten, dass die Viruspartikel beladen sind. Nicht mit Nukleinsäuren beladene Viruspartikel, d.h. leere Viruspartikel sind nicht funktionale Nebenprodukte und müssen entfernt werden.

Für den Nachweis der An- oder Abwesenheit von Nukleinsäuren in Viruspartikeln stehen verschiedene Methoden zur Verfügung. Dazu gehören PCR-Verfahren in Kombination mit ELISA-Verfahren, Cryo-Elekronenmikroskopie, dynamische Lichtstreuung (DLS), analytische Ultrazentrifugation oder mehrstufige Verfahren mit verschiedenen Biosensoren unter Verwendung der Biolayer-Interferometrie. Außerdem beeinflussen Nukleinsäuren die Ladung des Viruscapsids, so dass eine Trennung aufgrund ihrer Ladung in der Ionenaustauschchromatographie möglich ist.

Es werden ständig neue Verfahren, Hilfsmittel und Geräte entwickelt, um die Herstellungsmethoden und den Umgang mit Proteinen, insbesondere von Biopharmazeutika zu vereinfachen und damit kostengünstiger zu gestalten.

Die vorliegende Erfindung hat die Aufgabe, Verfahren zu entwickeln, mit denen Varianten eines Zielproteins, nämlich Antikörper, Fc-Fusionsprotein, Antikörperfragment, bi- und multispezifische Antikörper sowie Viruspartikel-basierte Therapeutika, Antikörper-Wirkstoff-Konjugate (AWK) und fehlerhaft assemblierte (z.B. fehlgepaarte oder fragmentierte) Proteine und weitere Nebenprodukte schnell detektiert werden können.

Der Nachweis der verschiedenen Nebenprodukte soll auf der Basis ihrer Hydrophobizität erfolgen. Diese Messungen eignen sich besonders für den Nachweis von fehlerhaft assemblierten Proteinen und unterschiedlich konjugierten Antikörper-Wirkstoff-Konjugate.

Methoden und Geräte zur Proteindetektion und -quantifizierung anhand ihrer physikalischen Parameter sind bekannt.

Eine Methode, die häufig zur Charakterisierung und Reinigung von Proteinen und anderen biologischen Moleküle eingesetzt wird, ist die Säulenchromatographie. Sie kann sowohl unter Normaldruck als auch unter erhöhtem Druck durchgeführt werden. Letztere erfordert oft teure Geräte wie HPLC oder FPLC.

Eine weitere Methode basiert auf der Verwendung von Biosensoren, wie beispielweise in US 5,804,453 B beschrieben.

Ein Biosensor ist eine Vorrichtung, die eine biologische Komponente mit einem physikalischen oder chemischen Messwandler kombiniert, um als Reaktion auf einen Zielanalyten ein messbares Signal zu erzeugen. Biosensoren können verschiedene physikalische Messprinzipien nutzen, um die Anwesenheit von Zielanalyten zu detektieren. Bekannt sind z.B. piezoelektrische Biosensoren, optische Detektionsverfahren, basierend auf Interferometrie oder Oberflächenplasmonenresonanz sowie kalorimetrische Messverfahren.

Biosensoren, die interferometrische Verfahren nutzen sind z. B. in US 5,804,453 B beschrieben. Biosensoren, die piezoelektrische Verfahren nutzen sind gleichfalls bekannt und z.B. in EP2017613 A1 beschrieben.

Biosensoren, die interferometrische Methoden verwenden werden besonders häufig in Kombination mit Mikrotiterplatten verwendet, weil die Biosensoren einfach herzustellen sind und aufgrund ihrer Form gut in die Näpfchen der Mikrotiterplatten getaucht werden können.

Die in der sogenannten Biolayer-Interferometrie verwendeten Biosensoren besitzen zwei reflektierende Oberflächen, an denen eine Phasenverschiebung gemessen wird, wenn sich Biomoleküle an der zweiten, weiter von der Lichtquelle entfernten Oberfläche anlagern.

Biosensoroberflächen müssen für die Bindung der Zielanalyte funktionalisiert werden. Häufig werden die Oberflächen mit Aminen funktionalisiert, um die weitere Kopplung von Proteinen oder Peptiden über eine Amidbindung zu realisieren und wie z.B. in WO 2008/033535 beschrieben.

Die Funktionalisierung erfolgt durch bifunktionelle Linker, die einerseits mit der Oberfläche des Biosensors reagieren und andererseits eine Funktionalität bereitstellen, die die Bindung des eigentlichen Bindemoleküls (z.B. eines Antikörpers) ermöglicht. Bei Glasoberflächen können dies z.B. Siloxane sein, eine klassische Methode für die Funktionalisierung von Glasoberflächen. Bei Polymeroberflächen können Copolymere aus z.B. Polystyrol und Di-vinylbenzol eingesetzt werden, die reaktiven Gruppen zur weiteren Funktionalisierung der Polymeroberflächen bereitstellen.

Während säulenchromatographische Methoden zum Nachweis und ggf. zur Identifizierung von unterschiedlich hydrophoben Proteinen verwendet werden, ist der Einsatz von Biosensoren bisher nicht bekannt.

Der Nachteil von säulenchromatographischen Methoden ist immer, dass vor der Analyse die Vorreinigung der Proben notwendig ist. Dies ist wichtig, da sonst das Säulenmaterial sehr schnell irreversibel inaktiviert wird und weil Fremdproteine durch die meistens verwendete UV-Absorptionsmessung bei z.B. 280 nm nicht vom Zielanalyt, hier Zielprotein, unterschieden werden können. Auch die Regeneration des Säulenmaterials zwischen den Läufen ist notwendig und zeitaufwendig.

Es erfolgt zunächst die Adsorption der Probe an eine sogenannte stationäre Phase (dem Säulenmaterial, das aus verschiedenen Materialien bestehen kann). Durch Zugabe von Laufmittel (mobile Phase(n)), d.h. Lösungen mit unterschiedlichen Eigenschaften (z.B. Salzgehalt) werden die hydrophoben Wechselwirkungen der Analyten mit der stationären Phase abgeschwächt, die adsorbierten Moleküle desorbiert und eluiert.

Wenn nichts anderes angegeben bezeichnet der Begriff "Salzgradient" die Veränderung der Salzkonzentration über einen bestimmten räumlichen und/oder zeitlichen Bereich.

Bei der hydrophoben Interaktionschromatographie (HIC, hydrophobic interaction chromatography) werden hydrophobe Oberflächen verwendet, oft unter Verwendung von Salzgradienten. Bei dieser Chromatographiemethode wird anfangs eine hohe Salzkonzentration verwendet, die die Hydrathülle der Proteine stört. Dadurch werden hydrophobe Bereiche der Proteine für hydrophobe Wechselwirkungen zugänglich. Diese Bereiche binden dann an die hydrophob-funktionalisierte Oberfläche, hier das hydrophob-funktionalisierte Säulenmaterial. Durch Erniedrigung der Salzkonzentration rekonstituiert sich die Hydrathülle, d.h. die Zugänglichkeit der hydrophoben Stellen nimmt ab oder verschwindet, so dass die Proteine nicht mehr gebunden werden und in Abhängigkeit der verwendeten Salzkonzentration eluieren.

Aufgabe der vorliegenden Erfindung ist es Vorrichtungen und Verfahren bereitzustellen, mit denen Proteine, wie Biopharmazeutika, insbesondere Antikörper, Fc-Fusionsproteine, Antikörperfragmente, bi- und multispezifische Antikörper sowie Viruspartikel-basierte Therapeutika direkt und ohne vorherige Probenreinigung effizient und schnell qualitativ und quantitativ bestimmt werden können. Die Messung soll zudem einfach und vorzugsweise mit im Labor routinemäßig verfügbaren Mitteln durchführbar sein.

Die Erfinder haben nun die in den Patentansprüchen geschützten Verfahren und Verwendungen sowie Vorrichtungen gefunden, mit denen biologische Moleküle, insbesondere Proteine einschließlich Antikörper-Wirkstoff-Konjugate aufgrund ihrer unterschiedlichen Hydrophobizität charakterisiert und getrennt werden können. Damit hat der Anwender die Möglichkeit schnell und unkompliziert unterschiedliche Proteine (z.B. Nebenprodukte bei der Proteinherstellung oder der Konjugation von Antikörpern mit Wirkstoffen) zu detektieren. Die Detektion erfolgt spektrometrisch (bevorzugt mittels Fluoreszenz-Spektrometrie) und/oder über Biosensoren. Die Nachteile der vorgenannten Verfahren (insbesondere langwierige Reinigungsprozesse vor der eigentlichen Analyse, Regenerierung von Material etc.) werden dadurch vermieden.

Die Erfindung betrifft somit ein Verfahren zur Analyse von biologischen Molekülen, insbesondere Proteinen einschließlich Antikörper-Wirkstoff-Konjugaten, unterschiedlicher Hydrophobizität unter Verwendung von Mikrotiterplatten, deren Vertiefungen (Wells) zumindest teilweise unterschiedliche Salzkonzentrationen aufweisen und unter Verwendung von hydrophob-funktionalisierten Oberflächen.

In einer Ausführungsform A betrifft die Erfindung ein Verfahren zur Analyse von Proteinen unterschiedlicher Hydrophobizität mittels Spektroskopie oder unter Einsatz eines Biosensor, umfassend die Verwendung von Mikrotiterplatten, in deren Vertiefungen (Wells) bei der Analyse zumindest teilweise unterschiedliche Salz-Endkonzentrationen vorhanden sind, deren Abstände im Bereich von 10 bis 500 mMol/L liegen, und unter Verwendung von hydrophob-funktionalisierten Oberflächen.

In einer Ausführungsform B betrifft die Erfindung ein Verfahren nach Ausführungsform A, wobei die Proteine therapeutisch wirksame Proteine sind ausgewählt aus Antikörpern, Fc-Fusionsproteinen, Antikörperfragmenten, bi- und multispezifischen Antikörpern und Viruspartikel-basierten Therapeutika und Antikörper-Wirkstoff-Konjugaten.

In einer Ausführungsform C betrifft die Erfindung ein Verfahren nach einem der vorhergehenden Ausführungsformen, wobei die unterschiedlichen Salz-Endkonzentrationen in nebeneinander oder untereinander liegenden Wells vorliegen und so einen Stufengradienten bilden, in dem Reihen oder Spalten mit jeweils aufsteigenden oder absteigenden Salzgehalten vorhanden sind.

In einer Ausführungsform D betrifft die Erfindung ein Verfahren nach einem der vorhergehenden Ausführungsformen, unter Verwendung von Markermolekülen, die spezifisch an die zu analysierenden Proteine binden, wobei die Markermoleküle mono- oder polyklonale Antikörper oder davon abgeleitete Fragmente wie Nanobodies oder Single-Domain-Antikörper sowie Peptide und RNA- oder DNA-basierte Aptamere sind und diese gegebenenfalls gekoppelt mit Fluoreszenzfarbstoffen Fluoreszenzmarker bilden.

In einer Ausführungsform E betrifft die Erfindung ein Verfahren nach Ausführungsform D unter Verwendung hydrophob-funktionalisierter Partikel sowie der Markermoleküle, an die Fluoreszenzfarbstoffe gekoppelt sind und die Fluoreszenzmarker darstellen, wobei die Fluoreszenzemission der ungebundenen mit Fluoreszenzmarkern markierten Proteine gemessen wird.

In einer Ausführungsform F betrifft die Erfindung ein Verfahren nach Ausführungsform E unter Verwendung einer PAIA-Platte, in der einige oder sämtliche Wells der als Messkammern ausgebildet sind in denen sich eine Erhebung befindet, die so geformt ist, dass ihre Grundfläche mindestens 50% der Bodenfläche einer Messkammer bzw. eines Wells einnimmt und vorzugsweise eine quadratische Grundfläche aufweist, die sich nach oben hin zumindest geringfügig verjüngt und die Form einer vierseitigen Pyramide hat, wobei das obere Ende der Erhebung spitz, flach oder konvex ist und vorteilhafterweise weist das obere Ende der Erhebung einen Durchmesser von weniger als 50 µm auf, so dass sich dort keine Versuchskomponenten ablagern, wobei die Höhe der Erhebung mindestens 10 % und höchstens 50 % der Höhe der Meßkammer bzw. des Wells beträgt und der Boden der Messkammer bzw. des Wells bis auf den Boden der Erhebung lichtundurchlässig ist, wobei die Erhebung lichtdurchlässig ist und ein Messfenster bildet, das zur Messung der Fluoreszenzemission der ungebundenen mit Fluoreszenzmarkern markierten Proteine verwendet wird.

In einer Ausführungsform G betrifft die Erfindung ein Verfahren nach Ausführungsform F, umfassend die folgenden Schritte:
a. Verwendung der Mikrotiterplatte, in deren Vertiefungen (Wells) zumindest teilweise unterschiedliche Salzmengen vorhanden sind, deren Salz-Endkonzentrationen so beschaffen sind, dass bei der Messung zumindest teilweise Salz-Endkonzentrationen vorhanden sind, deren Abstände im Bereich von 10 bis 500 mMol/L liegen;
b. Zugabe von je einem Aliquot ggf. mit Wasser verdünnten Probe der zu analysierenden Proteine in die vorbehandelten Wells;
c. Zugabe der hydrophob-funktionalisierten Partikel;
d. Zugabe eines oder mehrerer Fluoreszenzmarker, gemäß Anspruch 5;
e. Mischen;
f. Messen der Fluoreszenzemission der ungebundenen mit Fluoreszenzmarkern markierten Proteine und Auswerten durch Korrelation der Messwerte mit den in den Wells vorliegenden Salz-Endkonzentrationen.

Bei dem vorstehenden Verfahren können die Schritte b, c und d in beliebiger Reihenfolge durchgeführt werden. Insbesondere kann das Verfahren so durchgeführt werden, dass nach der Durchführung der Schritte a, c und/oder d die Platte einem Trocknungsschritt unterzogen wird. Nach dem Trocknen wird das Verfahren mit Schritt b fortgesetzt, gefolgt von den übrigen Schritten.

In einer Ausführungsform H betrifft die Erfindung ein Verfahren nach Ausführungsform G, wobei Schritte b, c und d in beliebiger Reihenfolge durchgeführt werden können.

In einer Ausführungsform I betrifft die Erfindung ein Verfahren nach Ausführungsform H, wobei in Schritt a eine Mikrotiterplatte verwendet wird, in der zusätzlich zu den unterschiedlichen Salzmengen noch hydrophob-funktionalisierte Partikel vorhanden sind, wobei dann Schritt c entfällt.

In einer Ausführungsform J betrifft die Erfindung ein Verfahren nach Ausführungsform H oder I, wobei in Schritt a eine Mikrotiterplatte verwendet wird, in der zusätzlich zu den Salzmengen noch Fluoreszenzmarker vorhanden sind, wobei dann Schritt d entfällt.

In einer Ausführungsform K betrifft die Erfindung ein Verfahren nach Ausführungsform I oder J, in dessen Vertiefungen (Wells) zumindest teilweise unterschiedliche Salzmengen vorhanden sind, die so bemessen sind, dass bei der Analyse in den entsprechend behandelten nebeneinander oder untereinanderliegenden Wells zumindest teilweise Salz-Endkonzentrationen vorhanden sind, deren Abstände im Bereich von 10 bis 500 mMol/L liegen, wobei in diesen Wells ferner hydrophob-funktionalisierte Partikel vorhanden sind und ggf. Fluoreszenzmarker gemäß Ausführungsform E vorhanden sind, wobei die vorgenannten Komponenten Salz, Partikel und Fluoreszenzmarker in trockener Form vorliegen.

In einer Ausführungsform L betrifft die Erfindung die Verwendung der Mikrotiterplatte nach Ausführungsform K zur Durchführung des Verfahrens nach Ausführungsform G, wobei die Schritte c und d entfallen.

In einer Ausführungsform M betrifft die Erfindung ein Verfahren nach einer der Ausführungsformen A bis C, unter Verwendung von Biosensoren, die hydrophob-funktionalisierte Oberflächen aufweisen und deren Form und Dimension dergestalt ist, dass sie in die Wells der Mikrotiterplatten eingeführt werden können.

In einer Ausführungsform N betrifft die Erfindung das Verfahren nach Ausführungsform M, wobei zusätzlich Markermoleküle vorhanden sind, insbesondere mono- oder polyklonale Antikörper oder davon abgeleitete Fragmente wie Nanobodies oder Single-Domain-Antikörper sowie Peptide und auf RNA- oder DNA-basierte Aptamere.

In einer Ausführungsform O betrifft die Erfindung ein Verfahren nach einer der Ausführungsformen M oder N umfassend die folgenden Schritte:
a. Verwendung der Mikrotiterplatte, in deren Vertiefungen (Wells) zumindest teilweise unterschiedliche Salzmengen vorhanden sind, deren Salz-Endkonzentrationen so beschaffen sind, dass bei der Messung zumindest teilweise Salz-Endkonzentrationen vorhanden sind, deren Abstände im Bereich von 10 bis 500 mMol/L liegen;
   ggf. Einstellen der gewünschten zumindest teilweise unterschiedlichen Salz-Endkonzentrationen in den Wells, wobei der oder die Abstände der Salzkonzentrationen im Bereich von 10 bis 500 mMol/L liegen;
b. Zugabe von je einem Aliquot ggf. mit Wasser verdünnten Probe der zu analysierenden Proteine in die vorbehandelten Wells;
c. Einführung des Biosensors in die behandelten und mit Probe versehenen Wells der Mikrotiterplatte;
d. Mischen;
e. Messen des ausgelösten Signals des Biosensors;
f. Korrelieren der Messung mit den in den Wells vorliegenden Salz-Endkonzentrationen, um die verschiedenen Hydrophobizitätsvarianten des zu bestimmenden Proteins zu ermitteln.

Schritt c im vorgenannten Verfahren kann auch wie folgt durchgeführt werden: Einführen der Biosensoren mit hydrophob-funktionalisierten Oberflächen in die Kavitäten mit dem Salzgradienten.

Schritt d und e des vorgenannten Verfahrens können zusammengefasst werden, nämlich durch Schütteln der Mikrotiterplatte, wodurch die Reaktion beschleunigt wird, und gleichzeitige (Echtzeit-) Messung der Bindung an die Oberfläche auf den Biosensoren.

Werden nicht aufgereinigten Proben, die Fremdproteine enthalten, verwendet oder wenn das Messsignal des Zielmoleküls zu niedrig ist, wird das Schütteln nach Erreichen des maximalen Messsignals beendet (d.h. Biosensoren sind gesättigt).

Nach Schritt e des vorgenannten Verfahrens können die weiteren Schritt e1 und e2 eingefügt werden, nämlich
e1. Entnehmen der Biosensoren aus der Mikrotiterplatte und Einführen in eine zweite Mikrotiterplatte gegeben, in deren Wells die gleichen Salz-Endkonzentrationen wie in eine zweite Titerplatte, die aber zusätzlich ein Markermolekül enthält, das spezifisch an das Zielmolekül bindet;
e2. Erneutes Schütteln der Mikrotiterplatte und gleichzeitige Messung der Bindung des Markermoleküls an die bereits an die hydrophob-funktionalisierten Oberflächen der Biosensoren gebundenen Zielproteine.
Die Bestimmung der Bindung des Zielproteins an die hydrophob-funktionalisierten Oberflächen in den entsprechenden Wells, erfolgt über das in Schritt e oder e1 gemessene Signal. Hierdurch erfolgt die Bestimmung von Proteinen unterschiedlicher Hydrophobizität.

Bei den hier beschriebenen Verfahren werden Salze oder Salzmischungen zur Herstellung des Salzgradienten verwendet. Die Mikrotiterplatte kann auch Wells ohne Beladung mit Salzgradienten aufweisen. Die endgültige Salzkonzentration wird spätestens bei der Probenbefüllung erreicht. Z. B. durch Verdünnen einer bestimmten Probenmenge mit einer entsprechenden Menge Flüssigkeit oder Puffer.Die Erfindung wird zur Analyse vor allem von Proteinen verwendet. Insbesondere von therapeutisch wirksamen Proteinen, wie Antikörpern und davon abgeleiteten Proteinformaten, wie Fc-Fusionsproteinen, Antikörperfragmenten bi- und mehrspezifische Antikörper sowie Viruspartikel-basierten Therapeutika und Antikörper-Wirkstoff-Konjugaten.

Bei den hydrophob-funktionalisierten Oberflächen handelt es sich vorzugsweise um hydrophob-funktionalisierte Partikel oder um Sensoren, wie Biosensoren, deren Messoberfläche so beschaffen ist, dass hydrophobe Proteine gebunden werden und deren Form und Dimension dergestalt ist, dass sie in die Wells der verwendeten Mikrotiterplatten eingeführt werden können.

Werden bei dem erfindungsgemäßen Verfahren Markermoleküle, wie z.B. mono- oder polyklonale Antikörper oder davon abgeleitete Fragmente wie Nanobodies oder Single-Domain-Antikörper sowie Peptide und auf RNA- oder DNA-basierte Aptamere verwendet, so weist das Zielprotein (z.B das Biopharmazeutikum) Bindungsstellen für ein oder mehrere Markermoleküle auf.

Die Verwendung von Markern, insbesondere Fluoreszenzmarkern in Kombination mit der PAIA-Platte - wie sie hier beschrieben ist - hat den Vorteil, dass andere Proteine, die aufgrund ihrer Hydrophobizität an die hydrophob-funktionalisierten Oberflächen binden können, aber nicht von dem oder den Markermolekülen gebunden werden, nicht detektiert werden. Solche Proteine sind z.B. Wirtszellproteine, wie sie typischerweise in Zellkulturüberständen vorliegen, oder auch Fehlassemblierungen bei der Herstellung von bi- oder mehrspezifischen Antikörpern, die keine Bindungsstelle für das oder die Markermoleküle (mehr) besitzen.

Markermoleküle sind Moleküle, die die biologischen Moleküle (z.B. Proteine) spezifisch binden können. Beispiele für Markermoleküle sind mono- oder polyklonale Antikörper, bzw. davon abgeleitete Fragmente, wie z.B. die sogenannten Nanobodies oder Single-Domain-Antikörper sowie Peptide und auf RNA- oder DNA-basierte Aptamere.

Die Markermoleküle können Farbstoffe, wie z.B. Fluoreszenzfarbstoffe aufweisen. Solche Marker werden nachfolgend Fluoreszenzmarker genannt. Die Farbstoffe können kovalent an die Fluoreszenzmarker gebunden sein oder z.B. über das wohlbekannte Streptavidin-Biotin-System, in dem ein fluoreszenzmarkiertes Streptavidin oder andere fluoreszenzmarkierte Biotinbindende Proteine wie Avidin oder Neutravidin an ein biotinyliertes Markermolekül gekoppelt wird.

Der Fachmann findet ohne großen technischen Aufwand heraus, welche Fluoreszenzmarker für das erfindungsgemäße Verfahren und die erfindungsgemäße Verwendung besonders geeignet sind. Beispiele für erfindungsgemäß verwendbare Fluoreszenzmarker sind mit Alexa 647-konjugierte Affibody der Firma Affibody AB oder Nano-Secondary^{®} alpaca anti-human IgG/anti-rabbit IgG, recombinant VHH, Alexa Fluor^{®} 647 (Art.-No. CTK0101 - Fa. ChromoTek GmbH)) Weitere Fluoreszenzmarker sind kommerziell verfügbar, z.B. bei den Firmen Jackson Immmunoresearch, oder Abcam. Diese sind bereits mit einem Fluoreszenzfarbstoff gekoppelt und können direkt verwendet werden. Alternativ können von diesen Firmen mono- oder polyklonale Antikörper gekauft und mit den verschiedenen kommerziell erhältlichen Farbstoffen gekoppelt werden, z.B. Fluorescein, sowie den Farbstoffen der Firmen Biotium, Atto-Tec, und ThermoFisher.

Bei der Auswahl der Fluoreszenzmarker ist darauf zu achten, dass der Fluoreszenzmarker bei keiner der zu verwendenden Assaybedingungen direkt an die hydrophob-funktionalisierten Oberflächen bindet. Dies ist für den Fachmann leicht herauszufinden, z.B. durch fluoreszenzmikroskopische Untersuchung von Mischungen der hydrophob-funktionalisierten Oberflächen mit dem Fluoreszenzmarker.

Die Anwendung des erfindungsgemäßen Verfahrens bezieht sich vor allem auf die Analyse, d.h. die Identifizierung und ggf. Quantifizierung von biologischen Molekülen, insbesondere von Proteinen unterschiedlicher Hydrophobizität. Dazu zählen z.B. Biopharmazeutika (d.h. therapeutisch wirksame Proteine), wie Antikörper, Fc-Fusionsproteine, Antikörperfragmente oder bi- und mehrspezifische Antikörper sowie Viruspartikel-basierte Therapeutika und Antikörper-Wirkstoff-Konjugate.

Bei den Proteinen unterschiedlicher Hydrophobizität kann es sich um das gleiche Protein (d.h. ein Protein mit gleicher Aminosäuresequenz) handeln, von dem jedoch mehrere Varianten mit unterschiedlicher Hydrophobizität vorhanden sind. Z.B. Antikörper, deren Hydrophobizität sich aufgrund unterschiedlicher Glykosylierung, Amidierung oder durch die Oxidation von Aminosäuren verändert hat.

Proteine mit unterschiedlicher Hydrophobizität sind auch solche, die fehlerhaft assembliert sind. Die fehlerhaft assemblierten Proteine besitzen unterschiedliche Aminosäuresequenzen bzw. unterschiedliche Arten von und ggf. Anzahl an Proteindomänen, wie z.B. leichte und schwere Ketten bei Antikörpern. Bei bispezifischen Antikörpern sind dies z.B. die sogenannten Homodimere oder Halfmere, die jeweils nur eine Art von schweren und leichten Ketten besitzen, obwohl solche bispezifischen Moleküle unterschiedliche leichte und schwere Ketten, mit denen unterschiedliche Bindungspartner gebunden werden können, aufweisen.

Proteine, die unterschiedliche Hydrophobizität aufweisen, sind auch unterschiedlich beladene Viruspartikel, wenn sie aufgrund ihrer Beladung mit Nukleinsäuren (DNA oder RNA), ihrer Glykosylierung, einer Amidierung und/oder Oxidation der Aminosäuren ihrer Kapsidproteine unterschiedlich hydrophob sind.

Im erfindungsgemäßen Verfahren mit spektrometrischer Auswertung werden Mikrotiterplatten verwendet. Es ist auch vorstellbar, dass statt der Mikrotiterplatten andere speziell präparierte Gefäße verwendet werden. Werden im erfindungsgemäßen Verfahren Fluoreszenzmarker eingesetzt, so wird eine PAIA-Platte, wie hier definiert, verwendet.

Ein besonderer Vorteil der Erfindung besteht darin, dass die Proben direkt (ggf. nach Verdünnung mit Wasser) gemessen werden können. Eine Aufreinigung vor Durchführung der Verfahren (der Assays) ist nicht erforderlich.

Wenn im erfindungsgemäßen Verfahren Biosensoren als hydrophob-funktionalisierte Oberflächen verwendet werden, kommen keine PAIA-Platten zum Einsatz.

Der Vorteil der Erfindung liegt darin, dass eine Vielzahl von Proben in relativ kurzer Zeit auf das Vorhandensein von unterschiedlichen Proteinen untersucht werden kann. Aufgrund der einfachen und auf Wunsch automatisierten Durchführung liegt in der Regel bereits nach weniger als 60 Minuten ein Messergebnis für mehr als 100 Proben gleichzeitig vor. Die für Mikrotiterplatten vorhandene Automatisierung (z.B. Pipettierroboter oder automatische Ausleseverfahren) kann genutzt werden.

Mikrotiterplatten sind bekannt und gewöhnlich sind sie rechteckig und haben eine unterschiedliche Anzahl von Vertiefungen, auch Wells, Näpfchen, Kavitäten oder Vertiefungen genannt. Diese Wells sind üblicherweise parallel zur Längsseite der Mikrotiterplatte in Reihen und parallel zur Schmalseite in Spalten angeordnet sind.

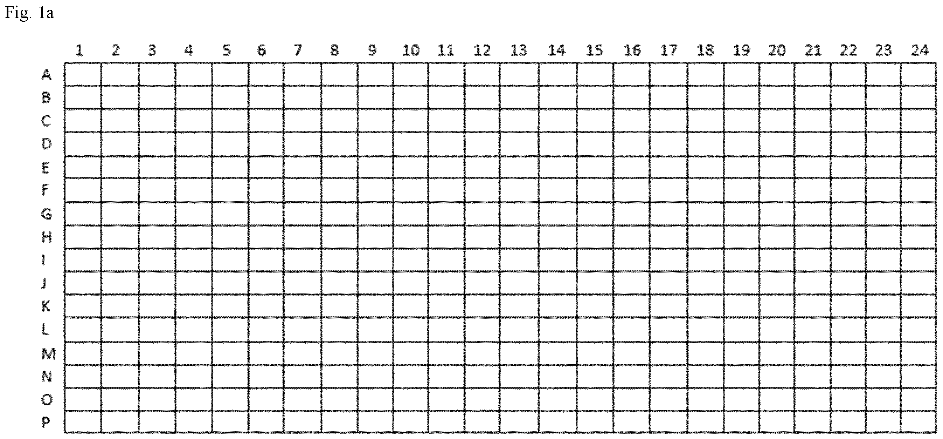

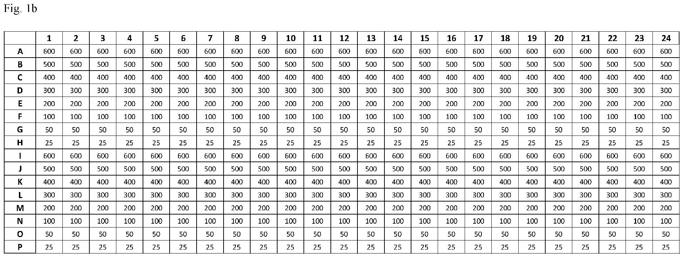

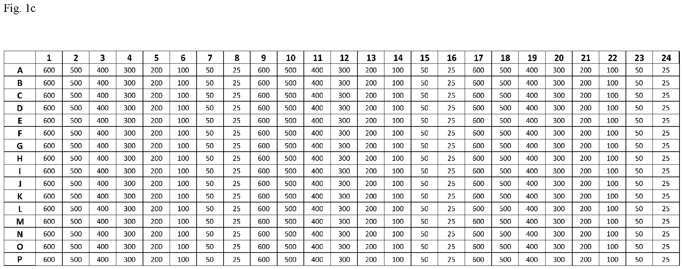

Für die Bezeichnung der Reihen werden in der Regel Buchstaben und für die Bezeichnung der Spalten Zahlen verwendet. Die Näpfchen einer Platte mit 384 Wells sind z. B. in 16 Reihen (von A bis P) und 24 Spalten (von 1 bis 24) angeordnet. Eine schematische Zeichnung einer solchen 384-Well-Platte ist in Fig. 1 dargestellt.

Die Wells einer 96-Well-Platte sind in 8 Reihen (A bis H) und 12 Spalten (1 bis 12) angeordnet. Dadurch kann jedes Well eindeutig identifiziert werden: So enthält die erste Reihe einer 96-Well-Platte 12 Wells, nämlich die Wells A1 bis A12. Die zweite Reihe enthält die Wells B1 bis B 12 usw.

Die spezielle Präparation der Mikrotiterplatte betrifft ein oder mehrere Wells oder im Falle der PAIA-Platte, wie unten näher beschrieben, ein oder mehrere Wells, die eine Messkammer bilden. Bei Verwendung von Mikrotiterplatten und einer spektrometrischen Auswertung sind die hydrophob-funktionalisierten Oberflächen bevorzugt hydrophob-funktionalisierte Partikel.

Die Wells der Mikrotiterplatte werden wie folgt präpariert:

In einem ersten Schritt wird die gewünschte Anzahl an Wells mit wässrigen Pufferlösungen befüllt, die unterschiedliche Salzkonzentrationen aufweisen - in den Beispielen auch Arbeitslösung genannt. Vorteilhafterweise weisen diese Salzlösungen den gleichen pH-Wert auf. Sie können aber auch unterschiedliche pH-Werte aufweisen.

Erfindungsgemäß geeignete Salze sind bekannt und werden auf die Proteine die analysiert werden sollen abgestimmt. Sie können vom Fachmann auch leicht ermittelt werden. Beispiele solcher Salze sind (NH₄)₂SO₄, NaH₂PO₄, Na₂HPO₄, Na₂SO₄, K₂SO₄, NaCl, KCl, MgCl₂, CaCL₂, MgSO₄ sowie Mischungen davon.

Die unterschiedlichen Salzkonzentrationen in der Arbeitslösung werden am einfachsten durch Mischen von zwei Puffern mit jeweils unterschiedlichen Salzkonzentrationen erhalten. Steht ein Puffer A ohne oder mit niedriger Salzkonzentration und ein Puffer B mit hoher Salzkonzentration zur Verfügung, kann die gewünschte Salzkonzentration durch Wahl eines geeigneten Mischungsverhältnisses eingestellt werden.

In der nachstehenden Tabelle ist beispielhaft ein 8-stufiges Salzgradientensystem (Salz-Endkonzentrationen) dargestellt, das mit (NH₄)₂SO₄ in (30 mM) TRIS-Puffer (pH 7,5) durch Verdünnen einer 2,4 Molaren (NH₄)₂SO₄-Lösung mit TRIS-Puffer erhalten wird. Dieses in Tabelle 1 dargestellte Gradientensystem ist besonders für die Ausführungsform des Verfahrens geeignet, in dem ein Trocknungsschritt vorgesehen ist

**Tabelle 1: Beispiel eines 8-stufigen Salzgradientensystems mit Angabe der Salzkonzentration der Arbeitslösung vor dem Entfernen der Lösung und der Salz-Endkonzentration in 60µL bei Versuchsdurchführung.**

| **Nr.** | 2,4M (NH₄)₂SO₄-Lösung, pH 7,5 | TRIS-Puffer, pH 7,5 [mL] | Arbeitslösung (NH₄)₂SO₄ in TRIS-Puffer pH 7,5 [Mol/L] | Gewünschte (NH₄)₂SO₄ - Endkonzentration [Mol/L] |
|---|---|---|---|---|
| | [mL] | | | |
| P1 | 875 | 125 | 2.1 | 0.7 |
| P2 | 750 | 250 | 1.8 | 0.6 |
| P3 | 625 | 375 | 1.5 | 0.5 |
| P4 | 500 | 500 | 1.2 | 0.4 |
| P5 | 375 | 625 | 0.9 | 0.3 |
| P6 | 250 | 750 | 0.6 | 0.2 |
| P7 | 125 | 875 | 0.3 | 0.1 |
| P8 | 0 | 1000 | 0 | 0 |

Als Puffersubstanzen können alle gängigen und bekannten Chemikalien verwendet werden, die Auswahl hängt von der Anwendung des jeweiligen Assays ab. Gebräuchliche Puffersysteme für die Handhabung von Proteinen sind z. B. PBS-Systeme (Phosphat-basierter saliner Puffer), Puffer aus der Gruppe der sogenannten "Good's buffer" wie z.B. TRIS (Tris(hydroxymethyl)aminomethan), MES (2-(N-morpholino)ethansulfonsäure), HEPES (4-(2-hydroxyethyl)-1-piperazinethansulfonsäure), Ammoniumacetat- oder Citratbasierte Puffer, etc.

Die im ersten Schritt des erfinderischen Verfahrens verwendete Salzkonzentration kann so gewählt werden, dass erst nach dem Einfüllen der für die Durchführung des Verfahrens benötigten Probelösung und ggf. weiterer Versuchskomponenten (wie z.B. Markermoleküle) die für den Versuch relevante Konzentration im Well erreicht wird. Diese relevante Konzentration wird im Folgenden auch Salz-Endkonzentration genannt. Die Salz-Endkonzentration wird im wesentlichen durch die Salzkonzentration der Arbeitslösung bestimmt. Die Salzmengen, die ggf. durch die Versuchskomponenten in die Wells eingebracht werden, sind gegenüber der Salzmenge und -konzentration der Arbeitslösung gering und werden normalerweise nicht zur Salz-Endkonzentration hinzugerechnet.

Die Unterschiede in der Salzkonzentration zwischen den Wells werden je nach den Anforderungen des Versuchs (Assay) variiert. Dies gilt auch für die Anzahl der Wells, über die sich der Assay erstreckt. Die Figuren 1b und 1c zeigen eine mögliche Belegung mit einem 8-Punkte oder 8-stufigen Salzgradienten.

Die Abstände der Salz-Endkonzentrationen sind so zu wählen, wie es die experimentelle Genauigkeit erfordert.

Die Besonderheit der Erfindung besteht vor allem darin, dass bei der Durchführung des Verfahrens in den in Reihen oder Spalten angeordneten Kavitäten der Mikrotiterplatte zumindest teilweise unterschiedliche Salz-Endkonzentrationen vorliegen.

Es ist vorteilhaft, wenn die Salz-Endkonzentrationsabstände in den einzelnen Wells im Bereich von 10 bis 500 mMol/L oder im Bereich von 20 bis 450 mMol/L oder im Bereich von 50 bis 400 mMol/L liegen. Bevorzugt im Bereich von 10 bis 250 mMol/L, 20 bis 200 mMol/L 10 bis 50 mMol/L, 20 bis 50 mMol/Lund 50 bis 100 mMol/L.

Sollen Proteine mit stark unterschiedlicher Hydrophobizität unterschieden werden, so ist es wichtig, mit dem Gradienten einen großen Salz-Endkonzentrationsbereich abzudecken. In diesem Fall ist es vorteilhaft, wenn der Abstand zwischen den Salz-Endkonzentrationen (Salzstufen) im Bereich von 50 mMol/L bis 100 mMol/L liegt. Diese Abstände der Konzentrationen (sog. Konzentrationsstufen) können gleich oder verschieden sein. Es hat sich gezeigt, dass bei Proteinen mit großen Unterschieden in der Hydrophobizität bereits acht verschiedene Salz-Endkonzentrationen ausreichen, um die Mischungen zu trennen (sog. 8-Stufen-Gradient, wie z.B. in Tabelle 1 dargestellt).

Wenn die Aufgabenstellung die Unterscheidung von Proteinen mit leicht unterschiedlicher Hydrophobizität erfordert, ist die Verwendung von Salz-Endkonzentrationen mit kleinerem Abstand von Vorteil. Solche kleinen Abstände liegen z. B. im Bereich von 10 bis 50 mMol/L oder im Bereich von 20 bis 50 mMol/L. Außerdem ist es dann von Vorteil, die Anzahl der Stufen zu erhöhen, um einen ausreichend großen Salzkonzentrationsbereich abzudecken.

Es hat sich für die Versuchsdurchführung als vorteilhaft herausgestellt, wenn die die Arbeitslösung mit unterschiedlichen Salzkonzentrationen in nebeneinander oder untereinander liegende Wells eingebracht wird, so dass Reihen oder Spalten mit jeweils auf- oder absteigendem Salzkonzentrationen und somit dann auch Salz-Endkonzentrationen vorliegen. Das ermöglicht eine vereinfachte Handhabung bei der Auswertung der Ergebnisse.

Selbstverständlich sollte die Belegung der Vertiefungen mit den zumindest teilweise verschiedenen Salzkonzentrationen der Arbeitslösung notiert werden. Dies kann separat oder auf der Platte erfolgen.

Nach Einfüllen der Arbeitslösungen enthaltend unterschiedliche Salzkonzentrationen, kann die Flüssigkeit (meistens Wasser) aus den Wells durch Trocknen entfernt werden, bevorzugt bei erhöhter Temperatur, d.h. einer Temperatur größer als 18 °C, bei Normaldruck oder unter vermindertem Druck. Die Trocknung hat den Vorteil, dass die so vorbereitete Mikrotiterplatte gelagert und aufbewahrt werden kann.

Bei der Verwendung der PAIA-Platte kann es für die Effizienz auch vorteilhaft sein, wenn auch der oder die Fluoreszenzmarker und/oder die hydrophob-funktionalisierten Oberflächen nach dem Einfüllen in die Wells getrocknet werden.

Die die Proteine mit unterschiedlicher Hydrophobizität enthaltende Probe wird nach der Entnahme gewöhnlich ohne weitere Behandlung (z.B. Aufreinigung) portionsweise in die Wells der Mikrotiterplatte, die auch eine PAIA-Platte sein kann, gegeben. Eine Verdünnung der Probe mit Wasser ist jedoch vorteilhaft, um eine den Versuch störende Konzentration an Puffersubstanzen zu erniedrigen. Die Menge und die Verdünnung der Probe wird so gewählt, dass die gewünschte Salz-Endkonzentration erreicht wird.

Das Portionieren geschieht manuell oder automatisch mit einem automatisierten Pipettierroboter (Liquidhandler).

Die während der Versuchsdurchführung in den Wells vorliegenden Salz-Endkonzentrationen sind entscheidend für die Bindungseigenschaften der jeweiligen Proteine mit unterschiedlicher Hydrophobizität an die hydrophob-funktionalisierte(n) Oberfläche(n).

Die Messung mit dem Biosensor oder - bei Verwendung der PAIA-Platte - vorzugsweise der Fluoreszenzintensität der ungebundenen Fluoreszenzmarker liefert einen Wert, aus dem auf die Hydrophobizität des biologischen Moleküls, insbesondere Protein geschlossen werden kann.

In einer besonderen Ausführungsform betrifft die Erfindung ein Verfahren zur Analyse von Proteinen unterschiedlicher Hydrophobizität unter Verwendung der speziell präparierten PAIA-Platten (d.h. in deren Wells sich zumindest teilweise unterschiedliche Salzkonzentrationen befinden, wie oben beschrieben) unter Verwendung von hydrophob-funktionalisierten Oberflächen, vorzugsweise hydrophob-funktionalisierten Partikeln, und Fluoreszenzmarkern als Marker. Die Detektion geschieht über Fluoreszenzmessung.

Die PAIA-Platte (auch PAIA-plate genannt) ist eine Mikrotiterplatte im SBS-Format mit einer bestimmten Anzahl an Wells Näpfchen (Wells), die als Messkammern ausgebildet sind, in denen die Erhebung (in der WO 2015/135840 A1 als Strukturelement bezeichnet) so geformt ist, dass ihre Grundfläche mindestens 50 % der Bodenfläche einer Messkammer bzw. eines Wells einnimmt. Sie sind bekannt und in WO 2015/135840 A1 beschrieben. Sie werden unter dem Handelsnamen PAIAplate 384 von der Firma PAIA Biotech GmbH hergestellt und vertrieben. Diese Platten haben 384 Wells.

Es versteht sich von selbst, dass die zu verwendende PAIA-Platte mehr oder weniger als 384 Wells haben kann.

Vorzugsweise weist die Erhebung eine quadratische Grundfläche auf, die sich nach oben hin (zumindest geringfügig) verjüngt und die Form einer vierseitigen Pyramide aufweist. Das obere Ende der Erhebung ist spitz, flach oder konvex, vorzugsweise läuft es spitz zu. Vorteilhafterweise weist das obere Ende der Erhebung einen Durchmesser von weniger als 50 µm auf, so dass sich dort keine Versuchskomponenten ablagern. Die Höhe der Erhebung beträgt mindestens 10 % und höchstens 50 % der Höhe der Meßkammer bzw. des Wells, vorzugsweise mindestens 15 % und höchstens 30 %. Der Boden der Messkammer bzw. des Wells ist bis auf den Boden der Erhebung lichtundurchlässig. Die Erhebung hingegen ist lichtdurchlässig und bildet ein Messfenster, das zur Messung der Fluoreszenzemission verwendet wird, wie in WO 2015/135840 A1 detailliert beschrieben.

Die PAIA-Platte ist insbesondere folgendermaßen ausgestattet: Die gewünschte Anzahl von Wells (einige oder alle) der Mikrotiterplatte sind als Messkammern ausgebildet, darin befindet sich eine Erhebung, die so geformt ist, dass ihre Grundfläche mindestens 50 % der Bodenfläche einer Messkammer bzw. eines Wells einnimmt und vorzugsweise eine quadratische Grundfläche, die sich nach oben hin zumindest geringfügig verjüngt und die Form einer vierseitigen Pyramide aufweist, wobei das obere Ende der Erhebung spitz, flach oder konvex ist und vorteilhafterweise weist das obere Ende der Erhebung einen Durchmesser von weniger als 50 µm auf, so dass sich dort keine Versuchskomponenten ablagern, wobei die Höhe der Erhebung mindestens 10 % und höchstens 50 % der Höhe der Meßkammer bzw. des Wells beträgt und der Boden der Messkammer bzw. des Wells bis auf den Boden der Erhebung lichtundurchlässig ist und die Erhebung ist lichtdurchlässig ist und ein Messfenster bildet, das zur Messung der Fluoreszenzemission verwendet wird.

Hinsichtlich der Messfenster wird auf die Beschreibung in der WO 2015/132840 A1 verwiesen und insoweit vollinhaltlich Bezug genommen. Zur Fluoreszenzmessung wird die erfindungsgemäße Messkammer bzw. Mikrotiterplatte von unten mit Anregungslicht bestrahlt, welches dann über die Erhebung in die Probe eingestrahlt wird. Um das Fluoreszenzsignal auszulesen, können handelsübliche Fluoreszenzreader verwendet werden.

Als Fluoreszenzreader können beispielsweise folgende Geräte erfindungsgemäß verwendet werden: Die Geräte der Serie Spectramax der Fa. Molecular Devices, die Systeme, der Fa. Tecan (Safire, der Infinite-Serie, oder SPARK), Reader der Fa. BMG Labtech (Omega, Clariostar, Pherastar) sowie Fluoreszenzmikroskope, z.B. der Fa. SynenTec (Cellavista und NyONE).

Die Erfindung unter Verwendung der PAIA-Platte kann insbesondere bei der Entwicklung von Zellkulturverfahren zur Herstellung von Proteinen, insbesondere therapeutisch wirksamen Proteinen, wie Antikörpern, Fc-Fusionsproteinen, Antikörperfragmenten, bi- und mehrspezifischen Antikörpern sowie Viruspartikel-basierten Therapeutika und Antikörper-Wirkstoff-Konjugaten vorteilhaft eingesetzt werden.

Die Erfindung betrifft daher auch die Verwendung von PAIA-Platten zur spektrometrischen Analyse, d.h. Detektion und Nachweis von Proteinen unterschiedlicher Hydrophobizität mittels Fluoreszenzspektroskopie, insbesondere von therapeutisch wirksamen Proteinen, wie Antikörpern, Fc-Fusionsproteinen, Antikörperfragmenten, bi- und mehrspezifischen Antikörpern sowie Viruspartikel-basierten Therapeutika und Antikörper-Wirkstoff-Konjugaten.

Die Erfindung betrifft auch eine PAIA-Platte, bei der in einer oder mehreren der Wells unterschiedliche Salzkonzentrationen vorhanden sind und in die hydrophob-funktionalisierte Partikel und gegebenenfalls Fluoreszenzmarker eingebracht sind. In einer vorteilhaften Ausführungsform sind die vorgenannten Versuchskomponenten in trockener Form in der PAIA-Platte vorhanden. Somit betrifft die Erfindung eine PAIA-Platte in der in den Wells der Platte mindestens in einer Reihe oder Spalte teilweise unterschiedliche ab- oder aufsteigende Salzmengen vorhanden sind. In gelöster oder trockner Form.

In einer Ausführungsform der Erfindung wird bei dem erfindungsgemäßen Verfahren eine Mikrotiterplatte verwendet, in deren Wells zumindest teilweise unterschiedliche Salzkonzentrationen vorhanden sind, und in der das erfindungsgemäße Verfahren mit Biosensoren durchgeführt werden, die hydrophob-funktionalisierte Oberflächen aufweisen.

Das erfindungsgemäße Verfahren zur quantitativen und/oder qualitativen Bestimmung von Proteinen unterschiedlicher Hydrophobizität unter Verwendung der PAIA-Platte kann wie folgt durchgeführt werden:
A. Zugabe von je einem Aliquot der Proteinprobe in die Vertiefungen (Wells) der Platte, wobei in den Wells eine zumindest teilweise unterschiedliche Salzkonzentration vorliegt;
B. Zugabe von hydrophob-funktionalisierten Partikeln;
C. Zugabe eines oder mehrerer Fluoreszenzmarker, die so ausgewählt sind, dass sie spezifisch an das zu bestimmende Protein binden;
D. gegebenenfalls Einstellen der gewünschten, zumindest teilweise unterschiedlichen End-Salzkonzentrationen mit gleichen oder unterschiedlichen Abständen im Bereich von 10 bis 500 mMol/L;
E. Mischen;
F. Messen der Intensität der ungebundenen Fluoreszenzmarker nach Absetzen der Partikel;
G. Auswertung durch Korrelation der Messwerte mit den in den Wells vorliegenden Salz-Endkonzentrationen.

Bei dem vorgenannten Verfahren können die Schritte A, B und C in beliebiger Reihenfolge durchgeführt werden. Werden Schritt B und/oder C - jeweils in beliebiger Reihenfolge - vor Schritt A durchgeführt, so kann nach Schritt B und/oder Schritt C ein Trocknungsschritt vorgesehen werden. Im Trocknungsschritt wird die mit den Versuchskomponenten eingebrachte Flüssigkeit entfernt, vorzugsweise durch Trocknen bei erhöhter Temperatur, wie hier beschrieben. Das Einstellen in Schritt D kann bereits durch Zugabe der in den Schritten A bis C genannten Versuchskomponenten erfolgen, z.B. unter Berücksichtigung des zugegebenen Gesamtvolumens der Versuchslösung.

Wird ein Trocknungsschritt eingefügt, so kann das Verfahren nach dem Trocknungsschritt beliebig lange unterbrochen und mit Zugabe der Probe, also Schritt A fortgesetzt werden. Das gilt auch für alle übrigen Verfahren, in denen ein Trocknungsschritt vorgesehen ist.

Die Erfindung betrifft eine PAIA-Platte, in deren Wells zumindest teilweise unterschiedliche Salzgehalte vorhanden sind, aus denen sich bei Versuchsdurchführung unterschiedliche Salz-Endkonzentrationen ergeben, wobei die Abstände der Salz-Endkonzentrationen gleich oder unterschiedlich sind den gleichen oder unterschiedlichen Abstand aufweisen, der im Bereich von 10 bis 500 mMol/L liegt. Die Platte kann zusätzlich noch hydrophob-funktionalisierte Oberflächen, vorzugsweise Partikel und/oder ggf. Fluoreszenzmarkern aufweisen, wobei die vorgenannten Versuchskomponenten gelöst oder getrocknet vorliegen.

Die Erfindung betrifft ferner ein sog. Kit of Parts umfassend die PAIA-Platte mit den vorgenannten Versuchskomponenten in getrocknetem Zustand.

Die Erfindung kann auch unter Verwendung von Biosensoren als hydrophob-funktionalisierte Oberflächen in Mikrotiterplatten durchgeführt werden. Das Verwenden einer PAIA-Platte ist dann nicht vorgesehen.

Deshalb betrifft die Erfindung auch ein Verfahren zur Analysevon Proteinen unterschiedlicher Hydrophobizität unter Einsatz von hydrophob-funktionalisierten Biosensoren und unter Verwendung von Mikrotiterplatten, in deren Wells zumindest teilweise unterschiedliche Salzgehalte vorhanden sind, aus denen sich bei Versuchsdurchführung unterschiedliche Salz-Endkonzentrationen ergeben, wobei die Salz-Endkonzentrationen den gleichen oder unterschiedlichen Abstand aufweisen, der im Bereich von 10 bis 500 mMol/L liegt.

Der Nachweis von Proteinen unterschiedlicher Hydrophobizität mittels Biosensoren kann erfolgen, indem hydrophob-funktionalisierte Oberflächen auf Biosensoren aufgebracht werden, die in die wie oben beschriebene vorbehandelten Wells von Mikrotiterplatten (keine PAIA-Platten), eingebracht werden und an denen die Bindung der Zielproteine gemessen wird.

Dazu werden vor allem optische Methoden wie die so genannte Biolayer-Interferometrie eingesetzt, bei der eine Phasenverschiebung des Lichts auftritt, wenn Moleküle an die Oberfläche des Biosensors binden. Diese Phasenverschiebung ist messbar und hängt von der Anzahl und Masse der an die Oberfläche gebundenen Moleküle ab.

Die Messung mit Biosensoren kann gleichzeitig, d.h. mit vielen Biosensoren erfolgen. Alternativ kann sie sequentiell erfolgen, d.h. ein Biosensor wird nacheinander in die verschiedenen Kavitäten der Mikrotiterplatte enthaltend eine unterschiedliche Salz-Endkonzentration eingeführt und muss vor jeder Messung eines neuen Wells regeneriert und gewaschen werden.

Die Regeneration von Biosensoren ist ein bekanntes Verfahren. Moderne Geräte sind in der Lage, mit mehreren Biosensoren gleichzeitig in verschiedenen Wells zu messen und damit einen wesentlich höheren Probendurchsatz zu erreichen.

Wenn bei der Messung nur kleine Mengen eines Proteins gebunden oder sehr kleine Proteine gemessen werden und deshalb nur ein sehr schwaches Messsignal erzeugt wird, ist es bei Biosensoren üblich, nach der Bindung des Zielmoleküls an die Oberfläche zur Signalverstärkung ein spezifisch an das Zielmolekül bindender Marker, z. B. einen Antikörper mit ausreichend großer Molekülmasse, an die Oberfläche zu binden. Reicht auch dies zur Signalverstärkung nicht aus, kann auch noch ein weiterer Marker, (hier Binder genannt, z.B. ein sekundärer Antikörper), der spezifisch an den primären Antikörper bindet, an den Biosensor binden.

Der gleiche Ansatz wird verwendet, wenn bei der Bindung des Proteins weitere unerwünschte Moleküle und Proteine ("Nicht-Zielmoleküle") an die Oberfläche gebunden werden. Dies kann z. B. der Fall sein, wenn die Probe nicht gereinigt wurde und Fremdproteine enthält, die ebenfalls an die Oberfläche binden.

In diesem Fall ermöglicht die Verwendung spezifischer Markermoleküle, z. B. eines Antikörpers, der spezifisch an das Zielprotein bindet, die Messung einer Phasenverschiebung aufgrund der Bindung des Markermoleküls, und diese Bindung hängt nur von der Menge der an die Oberfläche gebundenen Zielmoleküle ab. Es handelt sich also um ein für das Zielmolekül spezifisches Messsignal.

Die hydrophob-funktionalisierten Oberflächen, insbesondere die Partikel im Falle der Verwendung einer PAIA-Platte, werden bei dem erfindungsgemäßen Verfahren so gewählt, dass sie Proteine unterschiedlicher Hydrophobizität binden können.

Bevorzugt weisen die Partikel mit hydrophob-funktionalisieren Oberflächen mittlere Durchmesser im Bereich von etwa 20 bis 200 µm auf. Bevorzugt weisen die Partikel mittlere Durchmesser im Bereich von 5 bis 100 µm oder 1 bis 70 µm auf.

Erfindungsgemäß verwendbar sind alle hydrophob-funktionalisierten Oberflächen und Partikel, wie sie standardmäßig für Verfahren wie die hydrophobe Interaktionschromatographie (HIC) eingesetzt werden. Derartige Oberflächen insbesondere Partikel sind bekannt. Die Oberflächen und Partikel beinhalten oft unpolare organische kurzkettige Kohlenwasserstoffe, wie z.B. (C₃-C₆)-Alkyl-Gruppen, Phenyl-, Propylgruppen sowie Ether- oder Amid-Gruppen. Geeignete Oberflächen sind auch solche, die in der Umkehrphasenchromatographie als stationäre Phasen verwendet werden, vor allem solche, mit langen Kohlenwasserstoffketten, z.B. (C₈-C₁₈)-Alkyl-Gruppen.

Geeignete hydrophob-funktionalisierte Partikel sind z.B. TOYOPEARL Ether, TOYOPEARL PPG, TOYOPEARL Phenyl, TOYOPEARL Butyl-600M, TOYOPEARL-SuperButyl, wie sie von der Firma Tosoh vertrieben werden. Gleichfalls geeignet ist Phenyl Sepharose^{™} 6 Fast Flow (high sub), Butyl Sepharose^{™} 4 Fast Flow, Octyl Sepharose^{™} 4 Fast Flow resin Butyl-S Sepharose^{™} 6 Fast Flow Phenyl Sepharose^{™} High Performance, sowie Capto^{™} Phenyl (high sub) resin Capto^{™} Butyl Capto^{™} Octyl, Capto^{™} Butyl-S, wie sie von der Firma Cytiva vertrieben werden. Ferner POROS^{™} Benzyl Ultra Hydrophobie Interaction Chromatography (HIC) Resin, POROS^{™} Ethyl Hydrophobie Interaction Chromatography (HIC) Resin, wie sie von der Firma ThermoFisher vertrieben werden.

### Beschreibung der Figuren:

Fig. 1a zeigt eine schematische Beschriftung der in einer 384-Wells Mikrotiterplatte vorhandenen Vertiefungen (Wells).
Fig. 1b zeigt eine schematische Darstellung einer 384-Well-Platte mit Angabe der Salz-Endkonzentrationen in mMol/L, wie sie in Beispiel 1 verwendet wird. Die unterschiedlichen Salz-Endkonzentrationen in A1 bis H1 und 11 bis P1 entsprechen jeweils einem 8-stufigen abnehmenden Salzgradienten, was sich in den Spalten 2 bis 24 wiederholt.
Fig. 1c zeigt eine schematische Darstellung einer 384-Well-Platte mit Angabe der Salz-Endkonzentrationen in mMol/L, wie sie in Beispiel 1 verwendet wird. Die unterschiedlichen Salz-Endkonzentrationen in A1 bis A8, A9 bis A16 und A17 bis A24 entsprechen jeweils einem 8-stufigen abnehmenden Salzgradienten, was sich in den Zeilen B bis P wiederholt.
Fig. 2 zeigt das Belegschema aus Beispiel 1, wobei pro Spalte unterschiedliche Proteinproben gemessen werden.
   BEV steht für: Bevacizumab (Handelsname Avastin).
   CET steht für Cetuximab (Handelsname Erbitux).
   DUP steht für Dupilumab (Handelsname Dupixent).
   TRA steht für Trastuzumab (Handelsname Herceptin).
Fig. 3 bezieht sich auf Beispiel 1 und zeigt auf der y-Achse die gemessenen Fluoreszenzintensitäten der ungebundenen Fluoreszenzmarker der Antikörper Bevacizumab, Cetuximab, Dupilumab und Trastuzumab bei der bei Messung im Wells vorhandenen Salz-Endkonzentration (x-Achse).
Fig. 4 bezieht sich auf Beispiel 2 und zeigt auf der y-Achse die Fluoreszenzintensitäten der ungebundenen Fluoreszenzmarker der Antikörpern Bevacizumab (BEV) und Ofatumumab (OFA) oder Mischungen davon, bei der bei Messung im Wells vorhandenen Salz-Endkonzentration (x-Achse).
Fig. 5 bezieht sich auf Beispiel 2 und zeigt die quasi-lineare Beziehung zwischen der Fluoreszenzintensität in einem Well mit der Salz-Endkonzentration 300 mMol/L und dem prozentualen Gehalt an Ofatumumab in den Proben (x-Achse).

### Beschriftung der Figuren

Fig. 1a: Schematische Beschriftung der in einer 384-Wells Platte vorhandenen Wells.

Fig. 1b: Schematische Darstellung einer 384-Well-Platte mit einem sich wiederholenden 8-stufigen Salzgradienten in einer Spalte.

Fig. 1c: Schematische Darstellung einer 384-Well-Platte mit einem sich wiederholenden 8-stufigen Salzgradienten in einer Zeile.

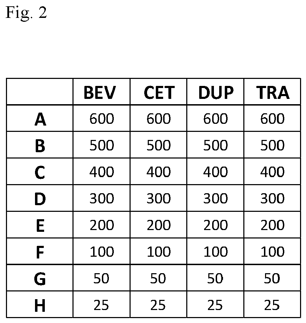

Fig. 2: Belegschema aus Beispiel 1.

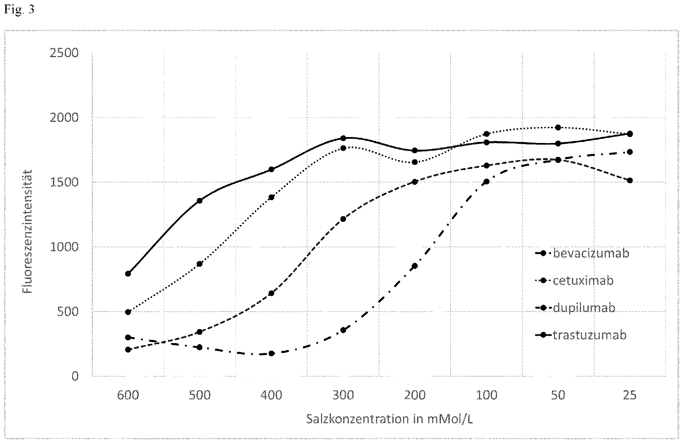

Fig. 3: Gemessene Fluoreszenzintensitäten der Proben aus Beispiel in Abhängigkeit der Salz-Endkonzentration.

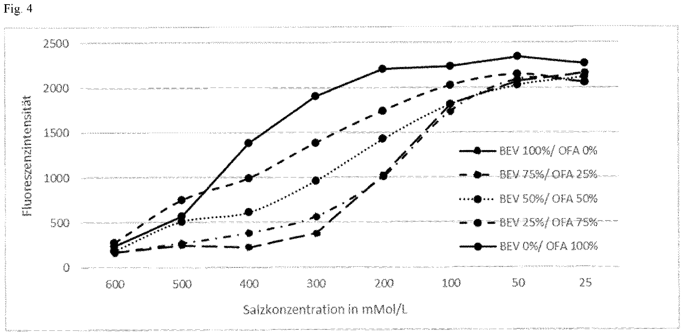

Fig. 4: Gemessene Fluoreszenzintensitäten von Antikörpern und Mischungen davon in Abhängigkeit der Salz-Endkonzentration.

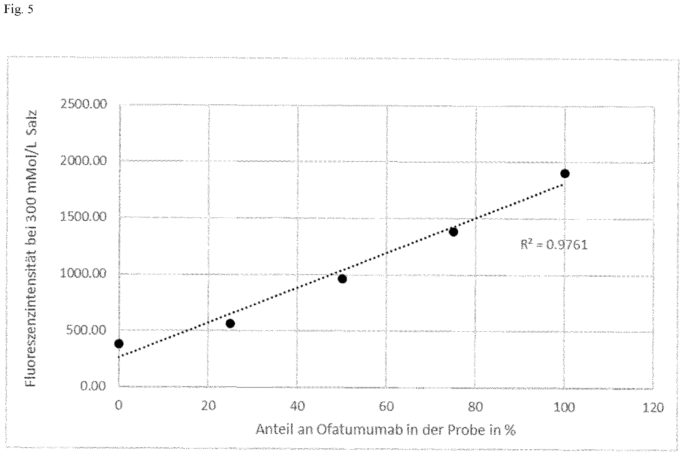

Fig. 5: Beziehung der Fluoreszenzintensität und dem Gehalt von Ofatumumab in einem Well bei einer Salzkonzentration von 300 mMol/L in verschiedenen Proben.

### Versuchsbeispiele:

Soweit nichts anderes angegeben, werden in den nachfolgenden Beispielen folgende Hilfsmittel und Reagenzien verwendet:

Als Reaktions- und Meßkammern werden die Wells (Näpfchen) einer PAIAplate 384 verwendet. Sie ist erhältlich von PAIA Biotech GmbH, und wird wie in WO 2015/135840 A1 hergestellt, verwendet. Das Arbeitsvolumen eines Wells beträgt 15µL-110µL.

Wenn nichts anderes erwähnt ist, wird als Puffer ein TRIS-Puffer mit pH 7,5 verwendet, enthaltend: 30 mM TRIS, 1,8 mM NaCl, 0,022% Tween 20, 1 mM CaCl₂ und 1 mM MgCl₂.

Als funktionalisierte Oberflächen werden folgende hydrophob-funktionalisierte Partikel verwendet, die Butylgruppen tragen: TOYOPEARL^{®} Butyl-600M Partikel (Fa. Tosoh, Art-Nr. 17546802) mit einem durchschnittlichen Durchmesser von ca. 65 µm, verwendet.

Soweit nichts anderes angegeben, werden die Messkammern vor der Messung wie folgt behandelt:

Vorbereitung der Messkammern / der Mikrotiterplatte:
(a) Herstellen einer 9 %ige Partikellösung bestehend aus TOYOPEARL^{®} Butyl-600M Partikel durch Verdünnen einer Stammlösung mit Wasser.
(b) Herstellen einer 9 %igen Saccharoselösung in Wasser als vorbeugender Schutz gegen das Austragen von Salzen oder anderen Versuchskomponenten während der Entfernung der flüssigen Bestandteile.
(c) Pipettieren von 30 µL der 9 %igen Partikellösung und 10 µL der 9 %igen Sucrose-Lösung in die entsprechenden Wells der PAIA-Platte.
(d) In jede dieser Wells werden 20 µL der Salz-Arbeitslösungen, d.h. einer gepufferten Salzlösung mit unterschiedlichen Salzkonzentrationen gegeben, wobei die Zugabe nach einem bestimmten Plan erfolgt, so dass nachvollzogen werden kann, in welcher Vertiefung sich welche Salzkonzentration befindet.
(e) Mischen und anschließendes Entfernen der Flüssigkeiten aus den Wells. Dafür wird die Platte für 48 Stunden bei 35 °C in einem Trockenschrank getrocknet.

Nach der Trocknung kann die so präparierte PAIA-Platte gelagert oder direkt für die Messung verwendet werden.

Sofern nicht anders angegeben, werden in den folgenden Beispielen die in Tabelle 2 aufgeführten unterschiedlich konzentrierten Salz-Arbeitslösungen P1 bis P8 hergestellt und als 8-stufiger Salzgradient verwendet.

Diese Arbeitslösungen werden durch Verdünnen einer 2,4 M (NH4)₂SO₄ -TRIS Stammlösung mit TRIS-Puffer gemäß der in Tabelle 2 angegebenen Verdünnungsreihe hergestellt.

**Tabelle 2: Verdünnungsreihe zur Herstellung unterschiedlicher Salzkonzentrationen in der Arbeitslösung für die Verwendung als 8-stufigen Salzgradient P1 bis P8.**

| (NH₄)₂SO₄-Lösung 2,4 M in TRIS-Puffer pH 7,5 [mL] | TRIS-Puffer [mL] | Konzentration der Arbeitslösung (NH₄)₂SO₄-Konzentration [Mol/L] | |
|---|---|---|---|
| 750 | 250 | 1.8 | P1 |
| 625 | 375 | 1.5 | P2 |
| 500 | 500 | 1.2 | P3 |
| 375 | 625 | 0.9 | P4 |
| 250 | 750 | 0.6 | P5 |
| 125 | 875 | 0.3 | P6 |
| 62.5 | 937.5 | 0.15 | P7 |
| 31.25 | 968.75 | 0.075 | P8 |

### Beispiel 1: Messung der Hydrophobizität verschiedener Antikörper

Getestet werden folgende Antikörper:
BEV = Bevacizumab (Handelsname Avastin), final formulierter therapeutischer Antikörper, mit deionisiertem Wasser auf eine Konzentration von 10 µg/mL verdünnt.
CET = Cetuximab (Handelsname Erbitux), final formulierter therapeutischer Antikörper, mit deionisiertem Wasser auf eine Konzentration von 10 µg/mL verdünnt.
DUP = Dupilumab (Handelsname Dupixent), final formulierter therapeutischer Antikörper, mit deionisiertem Wasser auf eine Konzentration von 10 µg/mL verdünnt.
TRA = Trastuzumab (Handelsname Herceptin), final formulierter therapeutischer Antikörper, mit deionisiertem Wasser auf eine Konzentration von 10 µg/mL verdünnt.

In die entsprechenden Wells der PAIA-Platte, in denen die Salzkonzentrationen P1 bis P8 vorhanden sind, wird jeweils 10µL der Probelösung zugegeben. Die Belegung kann der Fig. 2 entnommen werden.

In diese Wells wird 50 µL des Fluoreszenzmarkers Nano-Secondary^{®} alpaca anti-human IgG/anti-rabbit IgG, recombinant VHH, Alexa Fluor^{®} 647 (Art.-No. CTK0101 - Fa. ChromoTek GmbH gegeben und alles gemischt.

Der Fluoreszenzmarker wird in einer Konzentration von 3 nM in einem PBS-Puffer (Phosphate buffered saline) mit 50 ppm BSA (Bovines Serum Albumin) verwendet.

Die Mikrotiterplatte wird bei Raumtemperatur bei 2200 rpm auf einem Eppendorf Thermomixer Comfort 30 Minuten lang geschüttelt und die Platte anschließend bei 500 x g eine Minute lang zentrifugiert.

Anschließend erfolgt die Messung der Fluoreszenzintensität der ungebundenen Fluoreszenzmarker von unten (bottom reading) in jeder befüllten Messkammer in einem Tecan Safire Fluoreszenzplattenreader bei einer Anregungswellenlänge von 630 nm und einer Emissionswellenlänge von 665 nm.

Die gemessenen Fluoreszenzintensitäten in Abhängigkeit der Salz-Endkonzentration sind in Fig. 3 dargestellt. Die Graphen in Fig. 3 zeigen also die unterschiedlichen Hydrophobizitäten der gemessenen Antikörper.

Bei einer hohen Salzkonzentration sind alle Antikörper an die hydrophob-funktionalisierten Partikel gebunden und damit auch die Fluoreszenzmarker, so dass die in der Lösung gemessene Fluoreszenzintensität niedrig ist. Wird die Salzkonzentration niedriger kommt es zu einer Ablösung der Antikörper von den Partikeln basierend auf deren Hydrophobizität.

So ist z.B. Bevacizumab bei der End-Salzkonzentration von 300 mM/L fast vollständig an die Partikel gebunden, während Dupilumab bei dieser Konzentration nur teilweise und Cetuximab und Trastuzumab nicht an die hydrophoben Partikel binden. Dies entspricht den Erwartungen aus der Literatur, in der Bevacizumab als am meisten hydrophob beschrieben wird (Jain et al.: Biophysical properties of the clinical-stage antibody landscape, PNAS (2017) 114 (5) 944-949).

### Beispiel 2: Messung an einer Mischung von Antikörpern unterschiedlicher Hydrophobizität

Als Fluoreszenzmarker wird der Nano-Secondary^{®} alpaca anti-human IgG/anti-rabbit IgG, recombinant VHH, Alexa Fluor^{®} 647 (Art.-No. CTK0101 - Fa. ChromoTek GmbH) verwendet. Dieser wird in einer Konzentration von 3 nM in einem PBS-Puffer (Phosphate buffered saline) mit 50 ppm BSA (Bovines Serum Albumin) verwendet.

Als Proteine werden die beiden monoklonalen Antikörper Bevacizumab (BEV) und Ofatumumab (OFA) jeweils einzeln sowie Mischungen dieser beiden Moleküle in den Mischungsverhältnissen 75:25, 50:50 und 25:75 verwendet. Die Gesamtkonzentration der Antikörper in allen Proben beträgt 10 µg/mL.

In die entsprechenden Wells der PAIA-Platte, in denen die Salzkonzentrationen P1 bis P8 (siehe Tabelle 2) vorhanden sind, wird jeweils 10µL der Probelösung zugegeben. Jede Probe wird in Triplikaten gemessen, so dass insgesamt 24 Wells pro Probe belegt werden. Danach wird in die Wells jeweils 50 µl der Fluoreszenzmarkerlösung gegeben.

Die Mikrotiterplatte wird bei Raumtemperatur bei 2200 rpm auf einem Eppendorf Thermomixer Comfort 30 Minuten lang geschüttelt und die Platte anschließend bei 500 x g eine Minute lang zentrifugiert.

Anschließend erfolgt die Messung der Fluoreszenzintensität der ungebundenen Fluoreszenzmarker von unten (bottom reading) in jeder Messkammer in einem Tecan Safire Fluoreszenzplattenreader bei einer Anregungswellenlänge von 630 nm und einer Emissionswellenlänge von 665 nm.

Das Diagramm in Fig. 4 zeigt die Verläufe der Fluoreszenzintensität der verschiedenen Proben in Abhängigkeit von der Salzkonzentration in den Wells.

Die Probe mit 100 % Bevacizumab (BEV) zeigt zwischen den Salz-Endkonzentrationen von 600 mM/L und 300 mM/L keinen Signalanstieg, während die anderen Proben in diesem Bereich eine mit zunehmendem Gehalt an Ofatumumab höhere Fluoreszenzintensitäten zeigen. Von der Fluoreszenzintensität bei der Salz-Endkonzentration von 300 mM/L ausgehend lässt sich daher der Gehalt an Ofatumumab in diesen Mischungen bestimmen.

Fig. 5 zeigt die quasi-lineare Beziehung zwischen der Fluoreszenzintensität in dem Well mit der Salz-Endkonzentration 300 mM/L und dem prozentualen Gehalt an Ofatumumab in den Proben.

## Patentansprüche

1. Verfahren zur Analyse von Proteinen unterschiedlicher Hydrophobizität mittels Spektroskopie oder unter Einsatz eines Biosensor, umfassend die Verwendung von Mikrotiterplatten, in deren Vertiefungen (Wells) bei der Analyse zumindest teilweise unterschiedliche Salz-Endkonzentrationen vorhanden sind, deren Abstände im Bereich von 10 bis 500 mMol/L liegen, und unter Verwendung von hydrophob-funktionalisierten Oberflächen.

2. Verfahren nach Anspruch 1, wobei die Proteine therapeutisch wirksame Proteine sind ausgewählt aus Antikörpern, Fc-Fusionsproteinen, Antikörperfragmenten, bi- und multispezifischen Antikörpern und Viruspartikel-basierten Therapeutika und Antikörper-Wirkstoff-Konjugaten.

3. Verfahren nach einem der vorhergehenden Ansprüche, wobei die unterschiedlichen Salz-Endkonzentrationen in nebeneinander oder untereinander liegenden Wells vorliegen und so einen Stufengradienten bilden, in dem Reihen oder Spalten mit j eweils aufsteigenden oder absteigenden Salzgehalten vorhanden sind.

4. Verfahren nach einem der vorhergehenden Ansprüche, unter Verwendung von Markermolekülen, die spezifisch an die zu analysierenden Proteine binden, wobei die Markermoleküle mono- oder polyklonale Antikörper oder davon abgeleitete Fragmente wie Nanobodies oder Single-Domain-Antikörper sowie Peptide und RNA- oder DNA-basierte Aptamere sind und diese gegebenenfalls gekoppelt mit Fluoreszenzfarbstoffen Fluoreszenzmarker bilden.

5. Verfahren nach Anspruch 4 unter Verwendung hydrophob-funktionalisierter Partikel sowie der Markermoleküle, an die Fluoreszenzfarbstoffe gekoppelt sind und die Fluoreszenzmarker darstellen, wobei die Fluoreszenzemission der ungebundenen mit Fluoreszenzmarkern markierten Proteine gemessen wird.

6. Verfahren nach Anspruch 5, unter Verwendung einer PAIA-Platte, in der einige oder sämtliche Wells der als Messkammern ausgebildet sind in denen sich eine Erhebung befindet, die so geformt ist, dass ihre Grundfläche mindestens 50% der Bodenfläche einer Messkammer bzw. eines Wells einnimmt und vorzugsweise eine quadratische Grundfläche aufweist, die sich nach oben hin zumindest geringfügig verjüngt und die Form einer vierseitigen Pyramide hat, wobei das obere Ende der Erhebung spitz, flach oder konvex ist und vorteilhafterweise weist das obere Ende der Erhebung einen Durchmesser von weniger als 50 µm auf, so dass sich dort keine Versuchskomponenten ablagern, wobei die Höhe der Erhebung mindestens 10 % und höchstens 50 % der Höhe der Meßkammer bzw. des Wells beträgt und der Boden der Messkammer bzw. des Wells bis auf den Boden der Erhebung lichtundurchlässig ist, wobei die Erhebung lichtdurchlässig ist und ein Messfenster bildet, das zur Messung der Fluoreszenzemission der ungebundenen mit Fluoreszenzmarkern markierten Proteine verwendet wird.

7. Verfahren nach Anspruch 6, umfassend die folgenden Schritte:
a. Verwendung der Mikrotiterplatte, in deren Vertiefungen (Wells) zumindest teilweise unterschiedliche Salzmengen vorhanden sind, deren Salz-Endkonzentrationen so beschaffen sind, dass bei der Messung zumindest teilweise Salz-Endkonzentrationen vorhanden sind, deren Abstände im Bereich von 10 bis 500 mMol/L liegen;
b. Zugabe von je einem Aliquot ggf. mit Wasser verdünnten Probe der zu analysierenden Proteine in die vorbehandelten Wells;
c. Zugabe der hydrophob-funktionalisierten Partikel;
d. Zugabe eines oder mehrerer Fluoreszenzmarker gemäß Anspruch 5;
e. Mischen;
f. Messen der Fluoreszenzemission der ungebundenen mit Fluoreszenzmarkern markierten Proteine und Auswerten durch Korrelation der Messwerte mit den in den Wells vorliegenden Salz-Endkonzentrationen.

8. Verfahren nach Anspruch 7, wobei Schritte b, c und d in beliebiger Reihenfolge durchgeführt werden können.

9. Verfahren nach Anspruch 8, wobei in Schritt a eine Mikrotiterplatte verwendet wird, in der zusätzlich zu den unterschiedlichen Salzmengen noch hydrophob-funktionalisierte Partikel vorhanden sind, wobei dann Schritt c entfällt.

10. Verfahren nach Anspruch 8 oder 9, wobei in Schritt a eine Mikrotiterplatte verwendet wird, in der zusätzlich zu den Salzmengen noch Fluoreszenzmarker vorhanden sind, wobei dann Schritt d entfällt.

11. Mikrotiterplatte wie in Anspruch 6 definiert zur Verwendung in einem der Verfahren 9 oder 10, in dessen Vertiefungen (Wells) zumindest teilweise unterschiedliche Salzmengen vorhanden sind, die so bemessen sind, dass bei der Analyse in den entsprechend behandelten nebeneinander oder untereinanderliegenden Wells zumindest teilweise Salz-Endkonzentrationen vorhanden sind, deren Abstände im Bereich von 10 bis 500 mMol/L liegen, wobei in diesen Wells ferner hydrophob-funktionalisierte Partikel vorhanden sind und ggf. Fluoreszenzmarker gemäß Anspruch 5 vorhanden sind, wobei die vorgenannten Komponenten Salz, Partikel und Fluoreszenzmarker in trockener Form vorliegen.

12. Verwendung der Mikrotiterplatte nach Anspruch 11 zur Durchführung des Verfahrens nach Anspruch 7, wobei die Schritte c und d entfallen.

13. Verfahren nach einem der Ansprüche 1 bis 3, unter Verwendung von Biosensoren, die hydrophob-funktionalisierten Oberflächen aufweisen und deren Form und Dimension dergestalt ist, dass sie in die Wells der Mikrotiterplatten eingeführt werden können.

14. Verfahren nach Anspruch 13, wobei zusätzlich Markermoleküle vorhanden sind, insbesondere mono- oder polyklonale Antikörper oder davon abgeleitete Fragmente wie Nanobodies oder Single-Domain-Antikörper sowie Peptide und auf RNA- oder DNA-basierte Aptamere.

15. Verfahren nach Anspruch 13 oder 14 umfassend die folgenden Schritte:
a. Verwendung der Mikrotiterplatte, in deren Vertiefungen (Wells) zumindest teilweise unterschiedliche Salzmengen vorhanden sind, deren Salz-Endkonzentrationen so beschaffen sind, dass bei der Messung zumindest teilweise Salz-Endkonzentrationen vorhanden sind, deren Abstände im Bereich von 10 bis 500 mMol/L liegen;
b. Zugabe von je einem Aliquot ggf. mit Wasser verdünnten Probe der zu analysierenden Proteine in die vorbehandelten Wells;
c. Einführung des Biosensors in die behandelten und mit Probe versehenen Wells der Mikrotiterplatte;
d. Mischen;
e. Messen des ausgelösten Signals des Biosensors;
f. Korrelieren der Messung mit den in den Wells vorliegenden Salz-Endkonzentrationen, um die verschiedenen Hydrophobizitätsvarianten des zu bestimmenden Proteins zu ermitteln.
